# EUROPEAN PATENT APPLICATION

(11) **EP 1 977 768 A1**
(43) Date of publication of application: **08.10.2008**
(21) Application number: 07007134.5
(22) Date of filing: 04.04.2007
(51) Int. Cl.: A61K 49/04, A61Q 11/00

(54) **Dental paste composition having X-Ray imaging property**

(71) Applicant: GC Corporation, Itabashi-ku Tokyo 174-8585 (JP)
(72) Inventor: Walsh, Laurence J., Ferny Hills, QLD 4055 (AU); Kato, Shinichi, Itabashi-ku Tokyo 174-8585 (JP); Kariya, Shuji, Itabashi-ku Tokyo 174-8585 (JP); Sato, Takuya, Itabashi-ku Tokyo 174-8585 (JP)
(74) Representative: Müller-Boré & Partner Patentanwälte

(57) **Abstract**

An objective of the present invention is to provide a dental paste composition having X-ray imaging property, which can be easily placed between teeth and then removed immediately after X-ray imaging, does not need a complicated process since it can be applied by a simple operation for a paste-like composition, and has excellent X-ray imaging properties even though applied in a small amount. The dental paste composition having X-ray imaging property comprises: (a) 3 to 25 wt% of polyhydric alcohol; (b) 0.5 to 10 wt% of a powdery high polymer material dissolved and swelled in the polyhydric alcohol and water; (c) 10 to 30 wt% of water; and (d) 25 to 80 wt% of a powdery X-rayimaging material. In the composition, the total weight of the polyhydric alcohol (a) and water (c) is 3 to 25 times of the weight of the powdery high polymer material (b). Further, to the composition can also be added (e) 0.01 to 5 wt% of a fluorine compound, and (f) 15 wt% or less of an additive which is one or more kinds selected from a cushion material, a sweetener, a colorant, a preservative, an antiseptic agent, a fungicide, a pH regulator, a thickener, an expander and a perfume.

## Description

The present invention relates to a dental paste composition having an excellent X-ray imaging property, which can be temporarily placed into a recessed part of a tooth cavity or an area of decalcification in order to easily confirm the presence of a small cavity at the time of a normal dental X-ray examination as part of conventional dental and medical treatment, and can be easily removed by washing with water or by using a dental instrument such as a scaler or the like after the X-ray imaging procedure is complete.

Diagnosis by X-ray imaging is a common and important diagnostic method which is used frequently in the dental field. Clinical examination with a dental mirror and probes is unable to detect many types of dental diseases which affect the teeth and their supporting tissues. The ability of dental Xrays to confirm early stages of disease is limited, and importantly they cannot reliably predict if an area of destruction has reached the point where a cavity has formed between teeth, for example molar teeth. The clinician must make an estimate in such cases but cannot be certain of their diagnosis. A false negative diagnosis, that is missing a cavity, will lead to undertreatment, with the possibility of complications from diseases or the dental pulp or endodontic infections, when a simple restoration placed at an earlier stage would have been indicated. On the other hand, a false positive diagnosis will lead the dentist to drill into the tooth structure and the place ofadental restoration, a process that weakens the tooth and can lead to sensitivity and pulp damage as complications.A non-cavitated area could have been arrested and reversed using current dental remineralizing technology such as casein phosphopeptides-amorphous calcium phosphates; in which case a dental restoration would not be necessary, and a sound tooth surface for the life of the patient would be ensured.

It is important that the dentists have as much information as possible to guide their decision to restore or not to restore a given tooth surface. This should be undertaken without the need to take additional Xrays, because of modern radiation hygiene which demands as few Xrays as possible should be used to achive the required information.

Contrast media which yield enhanced information are well established in medical radiology and particularly in vascular medicine, cardiology and neurosurgery. Other than for imaging of the major salivary glands in medical radiology clinics, the use of contrast agents to enhance the diagnostic yield of dental Xrays has not previously been described. This invention decribes a means to confirm the states of a decalcification or cavity formed by dental caries, by using a composition having X-ray contrast properties that is filled into the decalcification part or the cavity prior to performing an X-ray examination , before planning restorative dental procedures, for example at the time of a dental check-up appointment.

There are in existence compositions having X-ray imaging properties, which are used as adjuncts to restorative dental care, where the opaque agent shows the presence of overhangs of material or debris which remains behind. To assist in their reliable detection on dental Xrays, most dental filling materials incorporate an opaque agent to increase the visibility of dental work on Xrays . This assists in normal diagnosis, in recognizing work done by previous dentists, and is valuable for forensic purposes (determining identity of unknown deceased persons). Examples of dental materials to which radio-opaque agents have been added include a composition sold as a temporary sealant, temporary fillings and dressings, and temporary cements used in crown and bridgework. Additional examples include an eugenol cement, a fatty acid cement, a polycarboxylic acid cement, and the like), and a resin-based temporary sealant (for example, refer to Japanese Patent Application Laid Open No. 6-65020, 8-188516, 9-77625, 2002-275017).

However, the temporary sealant is made for the purpose of remaining for a short period (a few days or one week) in an unfinished cavity, to protect the dental pulp and occupy the space until a permanent dental filling can be completed. Such materials are soft when inserted and then harden after filling. Thus, there is a problem that it is difficult to remove the cured material from some areas of the mouth. If it were to, unknowingly, enter a small cavity on a tooth surface, it would be very difficult to detect that this had occurred, or to remove the excess, since even the tip part of a dental scaler could not enter such a site. There also is the problem that the manipulation properties of the conventional temporary sealant are not good. Further, there are following complicated aspects of their use. For example, it is necessary for the cement type sealant materials to mix a powder component and a liquid component, while it is necessary for the resin type to operate a photoirradiation apparatus in order to cure the resin. It is necessary for the thermoplastic resin to apply heat in order to soften the resin. Furthermore, it is most unlikely that any Xray imaging will be undertaken at the time of using these materials since their purpose relates to extended dental restorative procedures and not to undertaking an Xray examination for the purpose of detecting dental caries. In other words, while some dental materials have radio-opaque properties, these are incidental to their main clinical use. They would not allow normal X-ray imaging, even if used at the same time, to detect small cavities or areas of decalcification since their method of application, physical properties, and penetration into small cavities between teeth would be all unsuitable for such a use.

An objective of the present invention is to provide a dental paste composition having X-ray imaging properties, which can be easily removed after Xray imaging, does not need a complicated process since it can be filled by a simple operation for a paste-like composition, and has excellent X-ray imaging properties even though only a small amount is used.

The earnest work was carried out in order to solve the above-mentioned problems and, as a result of this, the followings were found out to complete the present invention. A dental paste composition having a suitable X-ray imaging property for detection of cavities on the proximal (interdental) surfaces of teeth contains: polyhydric alcohol; a powdery high polymer material which is dissolved and swelled in the polyhydric alcohol and water; water; and a powdery X-ray imaging material. Further, in the composition, the total weight of polyhydric alcohol and water has a specified ratio to the weight of the powdery high polymer material. In such a constitution, the dental paste composition can be quickly filled in a small cavity or decalcification site, such as between molar teeth, and can then be easily removed from the area or the cavity by washing with water or by using a dental instrument such as a scaler.

The present invention has the following aspect. A dental paste composition having X-ray imaging property includes:
(a) 3 to 25 wt% of polyhydric alcohol;
(b) 0.5 to 10 wt% of a powdery high polymer material dissolved and swelled in the polyhydric alcohol and water;
(c) 10 to 30 wt% of water; and
(d) 25 to 80 wt% of a powdery X-ray imaging material,
   where the total weight of the polyhydric alcohol (a) and water (c) is 3 to 25 times of the weight of the powdery high polymer material (b).

The dental paste composition can further include:
(e) 0.01 to 5 wt% of a fluorine compound; and
(f) 15 wt% or less of an additive,
where the additive is one or more kinds selected from a cushioning or fillermaterial, a sweetener, a colorant, a preservative, an antiseptic agent, a fungicide, a pH regulator, a thickener, an expander and a perfume.

In such a dental paste composition having X-ray imaging property, the powdery X-ray imaging material (d) is preferably one or more kinds selected from barium sulfate, calciumtungstate, strontiumoxide, zirconium oxide, bismuth oxide, lanthanum oxide, zinc oxide, zirconium phosphate, bismuth subcarbonate, bismuth sulfate, zirconium silicate, ytterbium fluoride, strontium glass, and barium glass. The polyhydric alcohol (a) is preferably one or more kinds selected from glycerol, diglycerol, propylene glycol, dipropylene glycol, butanediol, pentanediol, butylene glycol, ethylene glycol, diethylene glycol, and triethyleneglycol. The powdery high polymer material (b) dissolved and swelled in polyhydric alcohol and water is preferably one or more kinds selected from cellulose compounds of carboxymethylcellulose, sodium carboxymethylcellulose, calcium carboxymethylcellulose, methylcellulose, hydroxyethylcellulose, hydroxymethylcellulose and hydroxypropylcellulose.

The dental paste composition having X-ray imaging property according to the present invention has water, a soluble material and a powdery X-ray imaging material as primary components. Thus, the paste composition can be easily removed after Xray imaging by washing with water or by using a dental instrument such as a scaler or the like. It does not need a complicated process, and has excellent operatability. Further, the dental paste composition has excellent X-ray imaging properties even when it is used in a small amount, since it contains a high amount of the X-ray imaging agent.

Hereinafter, a dental paste composition having X-ray imaging property according to the present invention will be described.

The polyhydric alcohol being as the (a) component is necessary to prevent drying of the dental paste composition having X-ray imaging property, to have wettability, and to stabilize the dental paste composition having the X-ray imaging property for a long time. The polyhydric alcohol is preferably one or more kinds selected from glycerol, diglycerol, propylene glycol, dipropylene glycol, butanediol, pentanediol, butylene glycol, ethylene glycol, diethylene glycol, and triethylene glycol. The blending ratio of the polyhydric alcohol is 3 to 25 wt% of the whole of the dental paste composition having X-ray imaging property. If the ratio is less than 3 wt%, the X-ray imaging agent becomes separated within the dental paste composition having X-ray imaging property, so that the dental paste composition cannot be preserved for a long time. Further, if the ratio is more than 25 wt%, the blending ratio of the powdery X-ray imaging material being as the (d) component is relatively decreased, so that the X-ray imaging property is decreased.

The powdery high polymer material dissolved and swelled in polyhydric alcohol and water being as the (b) component is necessary to prevent precipitating of the powdery X-ray imaging material being as the (d) component by dissolving and swelling in the polyhydric alcohol being as the (a) component and water being as the (c) component. The powdery high polymer material is preferably one or more kinds selected from cellulose compounds of carboxymethylcellulose, sodium carboxymethylcellulose, calcium carboxymethylcellulose, methylcellulose, hydroxyethylcellulose, hydroxymethylcellulose and hydroxypropylcellulose. Theblendingratioofthe (b) material is 0.5 to 10 wt% of the whole of the dental paste composition having X-ray imaging property. If the blending ratio is less than 0.5 wt%, the viscosity of the dental paste composition having X-ray imaging property is too low, so that the composition easily flows out after placing into an interdental region (such as between molar teeth) or filling in a cavity. If the blending ratio is more than 10 wt%, the viscosity of the dental paste composition having X-ray imaging property becomes too high, so that the composition is hardly to apply into a small cavity or between the posterior teeth in the mouth.

Water as the (c) component is used as a medium for dissolving and swelling the powdery high polymer material being as the (b) component together with the polyhydric alcohol being as the (a) component, and is necessary to decrease stimulativeness of the polyhydric alcohol being as the (a) component. Further, water is necessary to decrease the necessary amount of the polyhydric alcohol being as the (a) component having high cost, where the required amount of the polyhydric alcohol is decreased since the solubility of the powdery high polymer material being as the (b) component in water is higher than that in the polyhydric alcohol being as the (a) component. The blending amount of water being as the (c) component is 10 to 30 wt% of the whole of the dental paste composition having X-ray imaging properties. If the blending ratio is less than 10 wt%, the viscosity of the dental paste composition having X-ray imaging property is too high, so that operatability is decreased, and the composition is difficult to apply into small areas in the oral cavity. If the blending amount is more than 30 wt%, the blending ratios of the other components are decreased, for example, the effect of the X-ray imaging material being as the (d) component may be decreased.

Further, it is necessary that the total weight of the polyhydric alcohol being as the (a) component and water being as the (c) component is 3 to 25 times of the weight of the powdery high polymer material being as the (b) component. The reason of this ratio is as follows. If the total weight of the polyhydric alcohol being as the (a) component and the water being as the (c) component, that is, the total weight of liquid components is less than 3 times of the weight of the powdery high polymer material being as the (b) component, the powdery high polymer material being as the (b) component is not fully dissolved and swelled in the liquid components. Or, even if the material (b) is fully dissolved and swelled, the viscosity of the dental paste composition having the X-ray imaging property is too high. Thus, the powdery X-ray imaging material being as the (d) component cannot be uniformly dispersed and mixed in the dental paste composition having X-ray imaging property.

The powdery X-ray imaging material being as the (d) component is necessary to give X-ray contrast imaging properties which is a main objective of the present invention. The powdery X-ray imaging material is one or more kinds selected from barium sulfate, calcium tungstate, strontium oxide, zirconium oxide, bismuth oxide, lanthanum oxide, zinc oxide, zirconium phosphate, bismuth subcarbonate, bismuth sulfate, zirconium silicate, ytterbium fluoride, strontium glass, and barium glass. 25 to 80 wt% of the powdery X-ray imaging material is blended in the dental paste composition having X-ray imaging properties. If the blending ratio is less than 25 wt%, proper X-ray contrast imaging properties cannot be obtained when the using amount of the dental paste composition having X-ray imaging property is small. If the ratio is more than 80 wt%, the preservation ability of the dental paste composition having X-ray imaging property for a long period of time is decreased.

The dental paste composition having X-ray imaging property according to the present invention can be blended with the fluorine compound being as the (e) component having an effect to prevent dental caries, in addition to the above-described components. When the dental paste composition having X-ray imaging property is applied on a tooth surface at the time of X-ray imaging, dental caries between teeth can be also prevented. That is, the dental paste composition having X-ray imaging properties according to the present invention could be used for treating a slight cavity which is generated by decalcification induced by dental caries on the tooth surface. Thus, if the fluorine compound is applied in the site of application of the paste, a cavity on the adjacent site could be prevented, while the cavity itself could be slowed or arrested. The blending ratio of the fluorine compound to be added is preferably 0.01 to 5 wt% in the dental paste composition having X-ray imaging properties, and more preferably 0.05 to 2 wt%. If the ratio is less than 0.01 wt%, the re-calcification effect for preventing dental caries gained by the fluoride ion is hardly worthwhile or significant. If the ratio is more than 5 wt%, it is not preferable from the point of safety to a human body, should some of the material be ingested, even though it is only present in the mouth for a very short period of time.

The dental paste composition having X-ray imaging property according to the present invention can be further constituted with additives being as the (f) component. The additive being as the (f) component can be various kinds of a cushioning or filler materials, a sweetener, a colorant, a preservative, an antiseptic agent, a fungicide, a pH regulator, a thickener, an expander and a perfume, which are conventionally used for a composition for an oral cavity.

Since the additive being as the (f) component is not a primary component for constituting the dental paste composition having X-ray imaging property according to the present invention, the blending ratio is necessarily 15 wt% or less. If the ratio is more than 15 wt%, the blending ratios of the other primary components are decreased, so that the original objective of the present invention may not be realized.

Hereinafter, Examples and Comparative examples are concretely described, but the present invention is not limited to the following examples.

### <Example>

A dental paste compositions having X-ray imaging property were produced for Examples and Comparative examples with the blending ratios shown in Table 1. The following tests were carried out so as to evaluate those compositions. Results were collectively shown in Table 1.

### <X-ray imaging property>

The test of X-ray imaging property was carried out referring to "JIS T 6609-1;2005 Water Based Cement-Part 1: Powdery liquid type acid and base based cement". That is, a polyester sheet was put under a lower part of a ring made of a fluororesin. The inner diameter of the ring is 15mm and thickness is 1mm. A testing body was filled in this ring and a polyester sheet was put on the testing body. Then, the testing body was pressed by a glass plate so as to be a testing piece. Further, human enamel is ground to have the thickness of 1 mm by a waterproof grinder, so as to be a testing body.

An X-ray film was put on a lead sheet having a thickness of 2 mm or more, and the testing piece and an aluminum step wedges (one step is 0.5 mm from the thickness of 0.5 mm to the thickness of 5 mm) were put on the X-ray film. Further, each testing body was arranged and placed on the lead sheet. Then, X-ray photographing was carried out under conditions of a distance from the X-ray film of 400 mm, tube voltage of 65 kV, current of 10 mA, and X-ray irradiating time of 0.3 seconds. The X-ray filmwas developed and fixed. The thickness of aluminum corresponding to the optical density of the testing piece image was comparatively evaluated from the optical density of this film by using a photographic densitometer. When the imaging property of the composition was equal to or less than that of the human enamel, the composition was judged not to be substantially used as the composition having X-ray imaging property. In addition, the X-ray imaging property of human enamel was 2.1 mm.

### <Preservation ability>

Each testing body was filled in a syringe for a composite resin, and preserved at 60 degree C and RH of 75%. After one week, a state of the paste was visually observed.

### <Removability from a cavity>

A cylindrical cavity having the diameter of 2 mm and the depth of 2 mm was formed in a human removed third molar, and each composition of Examples and Comparative examples was filled in the cavity. Then, the time for washing with water and removing the composition from the cavity was measured by a dental spray "Spray Bit 4000L: GC Corporation".

Clearly from Table 1, as for the dental paste compositions having X-ray imaging property according to the present invention shown with Examples 1 to 15, the X-ray imaging property was sufficiently higher than that of the human enamel, the preservation ability was sufficient, and the composition can be easily washed with water and removed after being used.

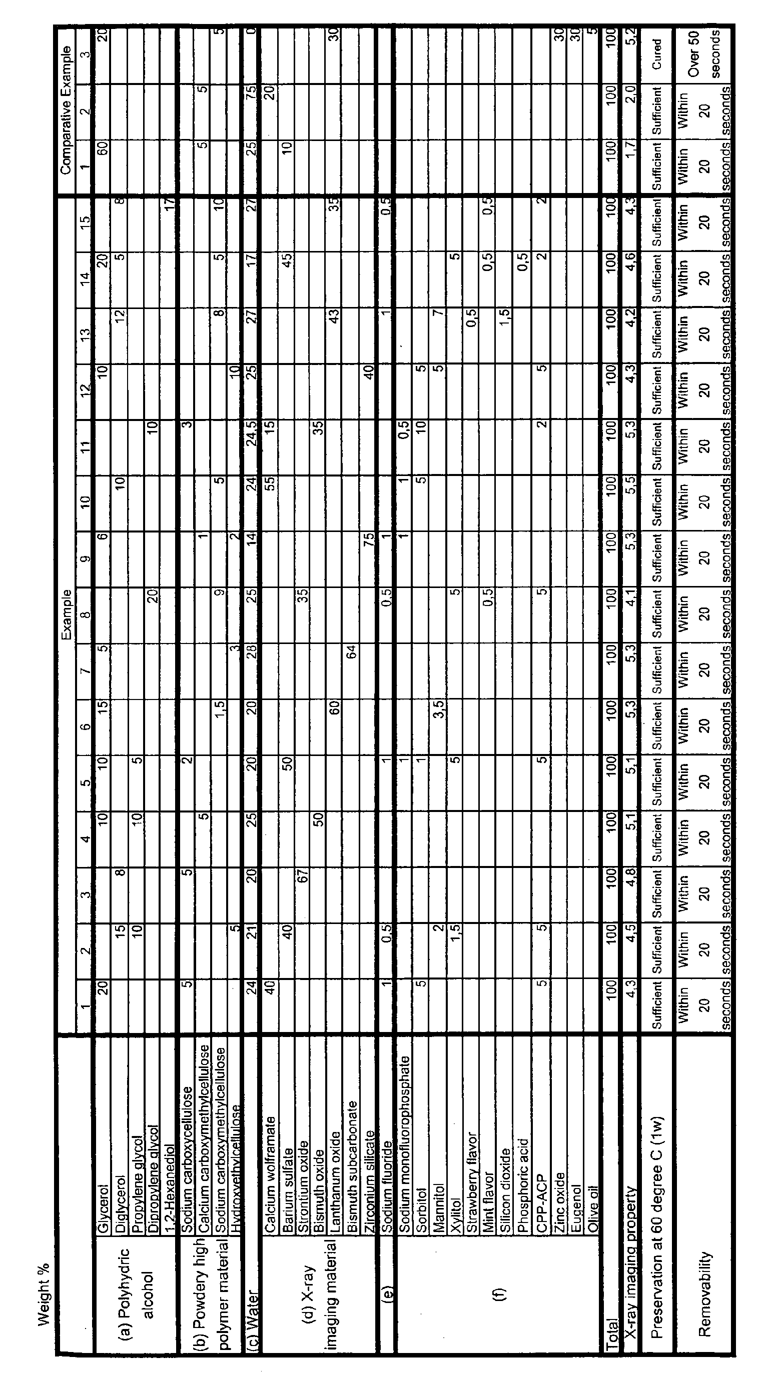

## Claims

1. A dental paste composition having X-ray imaging property, comprising:
(a) 3 to 25 wt% of polyhydric alcohol;
(b) 0.5 to 10 wt% of a powdery high polymer material dissolved and swelled in the polyhydric alcohol and water;
(c) 10 to 30 wt% of water; and
(d) 25 to 80 wt% of a powdery X-ray imaging material,
wherein the total weight of the polyhydric alcohol (a) and water (c) is 3 to 25 times of the weight of the powdery high polymer material (b).

2. The dental paste composition having X-ray imaging property as claimed in claim 1, the composition further comprising:
(e) 0.01 to 5 wt% of a fluorine compound.

3. The dental paste composition having X-ray imaging property as claimed in claim 1 or 2, the composition further comprising:
(f) 15 wt% or less of an additive.

4. The dental paste composition having X-ray imaging property as claimed in claim 3,
wherein the additive (f) is one or more kinds selected from a cushion material, a sweetener, a colorant, a preservative, an antiseptic agent, a fungicide, a pH regulator, thickener, an expander and a perfume.

5. The dental paste composition having X-ray imaging property as claimed in any one of claims 1 to 4,
wherein the powdery X-ray imaging material (d) is preferably one or more kinds selected from barium sulfate, calcium tungstate, strontiumoxide, zirconium oxide, bismuth oxide, lanthanum oxide, zinc oxide, zirconium phosphate, bismuth subcarbonate, bismuth sulfate, zirconium silicate, ytterbium fluoride, strontium glass, and barium glass.

6. The dental paste composition having X-ray imaging property as claimed in any one of claims 1 to 5,
wherein the polyhydric alcohol (a) is preferably one or more kinds selected from glycerol, diglycerol, propylene glycol, dipropylene glycol, butanediol, pentanediol, butylene glycol, ethylene glycol, diethylene glycol, and triethylene glycol.

7. The dental paste composition having X-ray imaging property as claimed in any one of claims 1 to 6,
wherein the powdery high polymer material (b) dissolved and swelled in polyhydric alcohol and water is preferably one or more kinds selected from cellulose compounds of carboxymethylcellulose, sodium carboxymethylcellulose, calcium carboxymethylcellulose, methylcellulose, hydroxyethylcellulose, hydroxymethylcellulose, and hydroxypropylcellulose.
